# EUROPEAN PATENT SPECIFICATION

(11) **EP 1 765 167 B1**
(45) Date of publication and mention of the grant of the patent: **14.04.2010**
(21) Application number: 05753095.8
(22) Date of filing: 14.06.2005
(51) Int. Cl.: A61B 5/20

(54) **DEVICE FOR MEASURING DATA RELATING TO URINE PRODUCTION OF A PATIENT**
VORRICHTUNG ZUR MESSUNG VON DATEN IN BEZUG AUF DIE URINPRODUKTION EINES PATIENTEN
DISPOSITIF DE MESURE DES DONNEES CONCERNANT LA PRODUCTION URINAIRE D'UN PATIENT

(30) Priority: 25.06.2004 NL 1026506
(43) Date of publication of application: 28.03.2007
(73) Proprietor: Pelvision B.V., 2629 HD Delft (NL)
(72) Inventor: DIJKMAN, Gerrat, NL-7524 RJ Enschede (NL)
(74) Representative: Mink-Lindenburg, Charlotte Hildegard
(86) International application number: PCT/NL2005/000431
(87) International publication number: WO 2006/001690

(56) References cited:
- EP-A- 0 342 028
- WO-A-2004/036343
- DE-A1- 3 541 649
- GB-A- 2 393 072
- US-A- 4 554 687
- US-A- 5 876 351

## Description

The invention relates to a device for measuring data relating to urine production of a patient for medical application, which device is provided with:
- a collecting device for collecting the urine of the patient;
- means for measuring the data; and
- a processor provided with memory means for storing the measurement data.

Urodynamic research has shown that many urological problems are caused by urinating incorrectly. It has been found in practice that a proper way to urinate can be taught by means of so-called urination training. Emphasis is laid here on relaxing the pelvic floor muscles during urination and on emptying the bladder as fully as possible. It is important to break the habit of incorrect urination at the youngest possible age, for which reason relatively large numbers of children are subjected to such urination training.

A device of the kind stated in the preamble is known in practice and is manufactured by the technical services department of the Academisch Ziekenhuis Utrecht (Utrecht Teaching Hospital). The known device comprises a toilet chair consisting of four legs and having thereon a toilet seat, a urine flow meter for placing under the toilet seat, and a computer connected to the urine flow meter by means of cabling. In practice a patient is admitted to hospital for some time for urination training. The urine which the patient excretes is for this purpose collected and guided along a urine flow meter. Important data such as the urine stream or flow are measured and stored on a computer for analysis by a medical expert. This training must then be continued at home by the patient using the known device.

The known device has the drawback of being awkward to place in the home situation. Most houses will in practice not have a room available in which the known device can be set up permanently. This usually means that the known device will have to be set up again each time it is used, which will make independent use thereof by children more difficult. In addition, the known device has a strange appearance and for this reason does not encourage children in particular to relieve themselves of their own accord.

It is an object of the invention to provide a device of the type stated in the preamble which obviates these drawbacks.

This object is achieved with a device as defined in claim 1, wherein according to the invention the urine collecting device is arranged for mounting on a toilet bowl. The device according to the invention is suitable for use on a normal toilet. The training can hereby take place in physiological manner in familiar surroundings which, particularly for the young user, has a stimulating effect. The simple operation alone of placing the collecting device in the existing toilet bowl suffices for use thereof, whereby the inconvenience for the user is reduced to a minimum.

According to the invention the processing unit is intended for reuse. The collecting device is a disposable article intended for once-only use, i.e. use by only one patient. A further advantage consists of the patient now only having to take the processing unit to the treating medical specialist for analysis of the measurement data.

A device for measuring data relating to urine production of a patient for medical application as described in the preamble provided with a urine collecting device that is arranged for mounting on a toilet bowl is known in the art and is for example described in EP 0 342 028 or US 4,554,687.

The known urine collecting device is suitable for use as a disposable article. Each time before use the disposable collecting device needs to be coupled to the processing unit. In order for the known device to function properly this coupling must be performed correctly, which may be difficult to achieve for many patients on their own at home. This coupling may be avoided by leaving the known disposable collecting device attached to the processing unit once coupled, but In this assembled state the known devices are rather bulky and difficult to handle.

It is an object of the invention to provide an improved device of the type stated above which obviates these drawbacks.

The device according to the invention is therefore characterized in that the processing unit can be mounted releasably on the collecting device.

In a practical embodiment hereof the collecting device is provided with a cover for at least partially receiving the processing unit. The cover allows coupling for the processing unit that can easily be performed by a patient at home.

The measuring means preferably comprise a flow sensor for measuring data relating to the urine flow from the patient.

In a compact preferred embodiment the flow sensor is incorporated in the processing unit.

According to a further preferred embodiment, the device further comprises an interface for connection to the processing unit for the purpose of displaying the measurement data. The user receives feedback during the training by means of the interface.

According to yet a further preferred embodiment, the processing unit is further provided with a transmitter for wireless transmission of the measurement data to the interface, this interface being provided with a receiver for receiving the measurement data. Wireless communication enhances convenience of use to a great extent.

In a further practical preferred embodiment of the device according to the invention, the urine collecting device is funnel-shaped and provided with means for mounting in the toilet bowl.

According to further elegant preferred embodiment, the urine collecting device takes an at least partially double-walled form and is provided with a liquid lock, and the measuring means are arranged to measure air displacement as a result of urine production. A reliable measurement can hereby be carried out using relatively inexpensive technical means.

The invention will now be elucidated in more detail hereinbelow with reference to the drawings, in which
Figure 1 is a schematic view of a preferred embodiment of the invention;
Figure 2 shows the device of figure 1 in exploded view;
Figures 3A and 3B show the device of figure 1 in cross-sectional view from two different viewpoints; and
Figure 4 shows schematically the operation of the device according to the invention.

Corresponding components are designated in the figures with the same reference numerals.

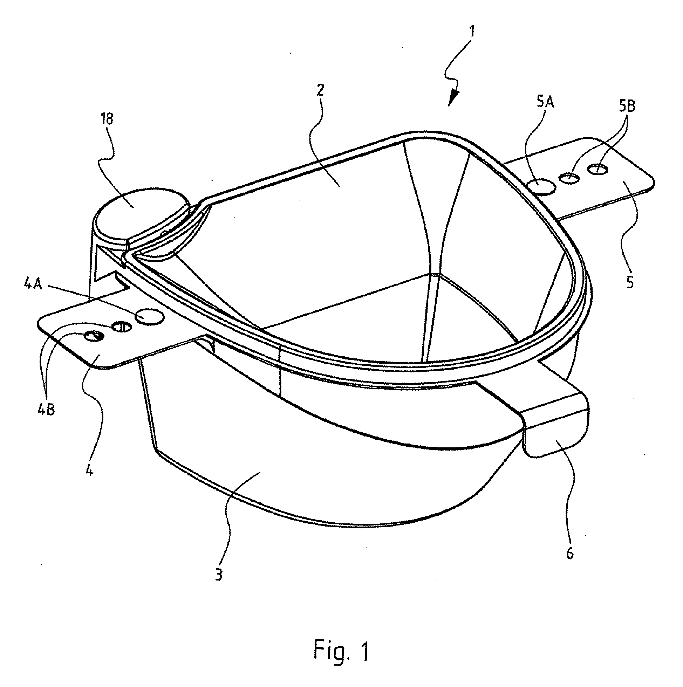

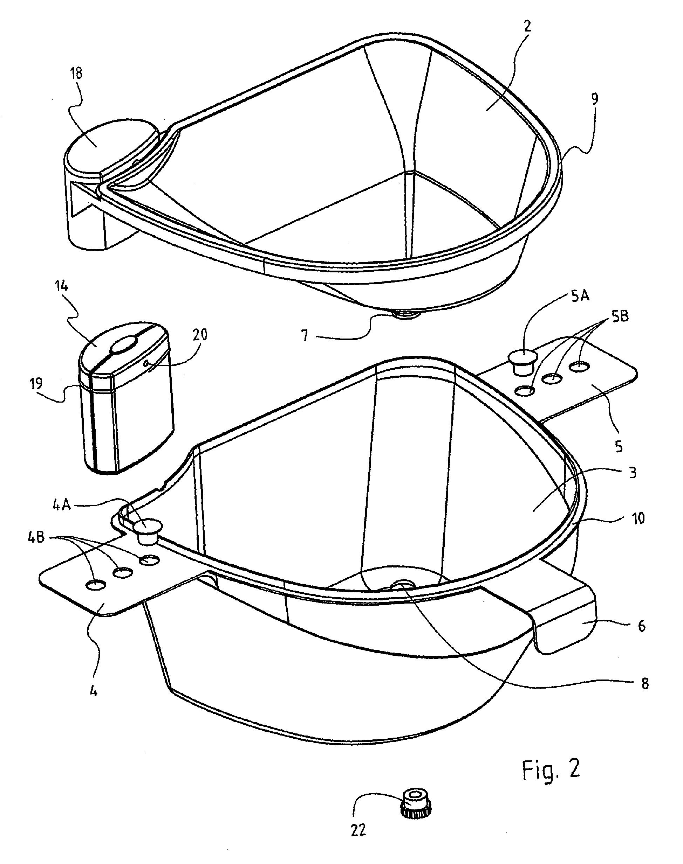

Figure 1 shows a schematic view of a device 1 according to the invention in a preferred embodiment. Figure 2 shows device 1 with exploded parts. Device 1 consists of two parts 2 and 3 which fit into each other. The dimensions of device 1 are such that the device can be placed in a toilet bowl. Part 3 is provided for this purpose with two lateral supports 4, 5 which rest on the edge of the toilet bowl during use. The supports can be made to fit toilet bowls of different dimensions by means of pin-hole connections 4A, 4B, 5A, 5B. Part 3 is further provided with a hook 6 which is placed round the front part of the toilet bowl during use. Hook 6 also serves as handgrip.

Part 2 is generally funnel-shaped and provided with an outlet opening 7. Part 3 has a larger volume and is provided with a closed neck 8 with upright edge 8A connecting onto outlet opening 7. Between opening 7 and edge 8A there is some clearance such that they co-act to form a liquid lock. It is noted that the length of the neck can be freely chosen. Both parts 2,3 are provided with rims 9, 10 respectively which fit into each other. Part 3 can be closed with a cap 22. The cap is preferably self-closing.

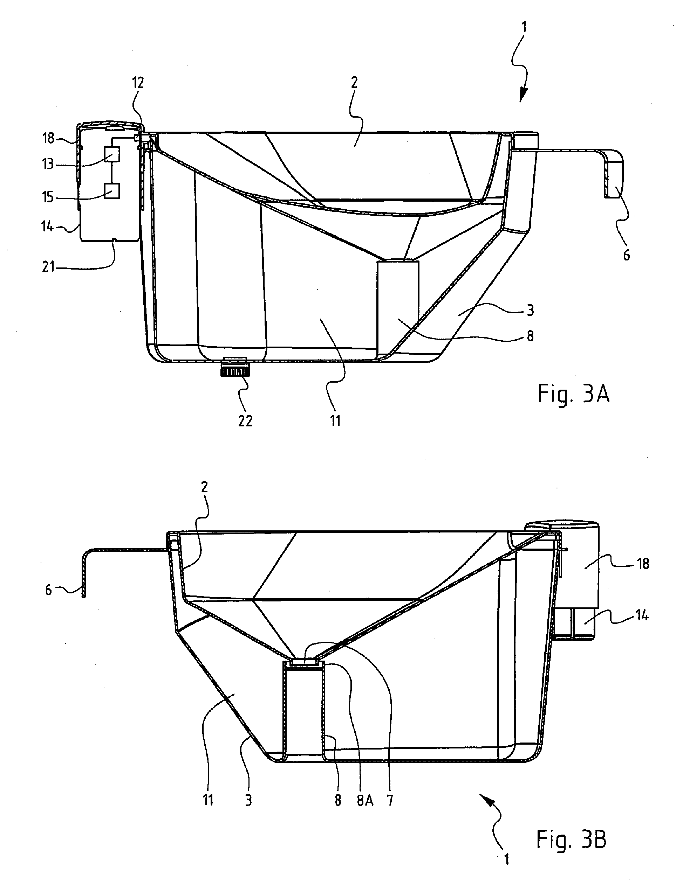

Figures 3A and 3B show device 1 in cross-sectional view from two different viewpoints. In this assembled position a volume 11 is defined between parts 2 and 3. The rims 9 and 10 seal this volume 11 airtightly.

A processor or processing unit 14 can be coupled to the collecting device. Processor 14 has for instance a housing which can be mounted on part 2. In the shown preferred embodiment part 2 is provided for this purpose with a cover 18 in which the housing of processor 14 can be partly received.

Processor 14 comprises measuring means 12 which are connected to memory means 13 for storage of relevant measurement data. Suitable measuring means are for instance a flow sensor, for instance the sensor of the AWM 43600 V type which is marketed by Honeywell. Suitable memory means are removable, such as a flash memory card.

Through the use of an O-ring 19 in the assembled position of collecting device 1 and processor 14 an air chamber is created around the top part of the processor at the position of opening 20 which provides access to measuring means 12. On the other side this air chamber communicates with volume 11.

Processor 14 is further provided with a transmitter 15 for transmitting the measurement data to an interface 16, this interface being provided with a receiver 17 for receiving the measurement data.

Prior to transmission the measurement data are preferably amplified and, if necessary, an A/D conversion takes place. Processor 14 comprises for this purpose suitable amplifier and conversion means. Transmitter 15 and receiver 17 are preferably suitable for wireless transmission/reception. Suitable frequencies for this purpose are approximately 4.33 or 868 MHz. Use can also be made of suitable commercially available systems such as BlueTooth or WiFi (Wireless Fidelity).

Interface 16 is intended for the display of the measurement data. A suitable interface comprises a processor or processing unit and a screen. An example of a suitable interface is a handheld computer such as a PDA (Personal Digital Assistant) or a game computer such as a Gameboy®. For display of the measurement data the interface has to be provided with software suitable for the purpose.

The processor comprises at least one connection 21 for an EMG electrode for measuring EMG signals of the patient. The EMG signals give an indication of the activity of the pelvic floor and of the abdominal pressure activity of the user, which are important additional data for the analysis of the measurement data by an expert.

According to the invention the processor 14 with accessories as well as interface 17 are intended for reuse. Collecting device 1 is a disposable article intended for once-only use, i.e. use by only one patient.

In the shown preferred embodiment the invention operates as follows. During use urine collected in collecting device 1 will flow out of part 2 via opening 7 into part 3. Due to the action of the liquid lock or water lock the urine will flow over edge 8A of neck 8 into volume 11. The air present in volume 11 is displaced as a result of the inflowing urine such that it passes flow sensor 12 via opening 20. Flow sensor 12 measures the air displacement and/or airflow as a measure of the urine flow. These measurement data are stored in memory 13. On the basis hereof, relevant data concerning urine production can be computed, such as the quantity of urine produced and the associated urine flow. Memory 13 can be read at any desired point in time to allow analysis of the measurement data by an expert. This can for instance form part of a visit to the treating medical specialist, such as the urologist.

After use the collecting device can be emptied by opening cap 22.

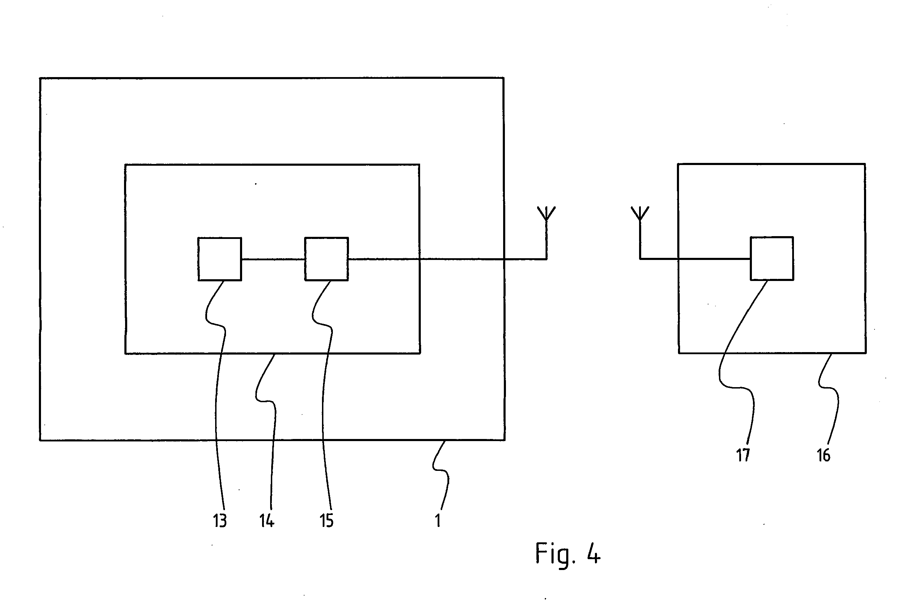

Figure 4 illustrates that the measurement data can then optionally be sent by means of transmitter 15 to receiver 17 of interface 16, where they can be shown to the user. This embodiment is particularly suitable for urination training in children, since direct feedback takes place during urination. By means of a second interface (not shown) the trainer can, if desired, receive the same information as the user. This direct feedback provides the trainer with the option of optimizing the training as much as possible.

It is noted, perhaps unnecessarily, that the invention is expressly not limited to the shown and described embodiment, but extends generally to any embodiment which falls within the scope of the appended claims, viewed in the light of the foregoing description and drawings.

## Claims

1. Device for measuring data relating to urine production of a patient for medical application, which device is provided with:
- a collecting device (1) for collecting the urine of the patient, which urine collecting device (1) is arranged for mounting on a toilet bowl;
- means (12) for measuring the data; and
- a processing unit (14) provided with memory means (13) for storing the measurement data, **characterized in that**,
the processing unit (14) is adapted to be mounted releasably on the collecting device (1).

2. Device as claimed in claim 1, wherein the collecting device (1) is provided with a cover (18) for at least partially receiving the processing unit (14).

3. Device as claimed in claim 1 or 2, wherein the measuring means comprise a flow sensor (12) for measuring data relating to the urine flow from the patient.

4. Device as claimed in claim 3, wherein the flow sensor (12) is incorporated in the processing unit (14).

5. Device as claimed in any of the preceding claims, further comprising an interface (16) for connection to the processing unit (14) for displaying the measurement data.

6. Device as claimed in claim 5, wherein the processing unit (14) is further provided with a transmitter (15) for wireless transmission of the measurement data to the interface (16), this interface being provided with a receiver (17) for receiving the measurement data.

7. Device as claimed in claim 5 or 6, wherein the interface (16) comprises a handheld computer, such as a PDA i.e. a Personal Digital Assistant.

8. Device as claimed in any of the preceding claims, wherein the urine collecting device (1) is funnel-shaped and provided with means (4, 4A, 5, 5A, 6) for mounting in the toilet bowl.

9. Device as claimed in any of the preceding claims, wherein the urine collecting device (1) takes an at least partially double-walled (2,3) form and is provided with a liquid lock (7, 8), and wherein the measuring means are arranged to measure air displacement as a result of the urine production.

## Patentansprüche

1. Vorrichtung zur Messung von Daten in Bezug auf die Urinproduktion eines Patienten zur medizinischen Anwendung, wobei die Vorrichtung mit Folgendem versehen ist:
- einer Sammelvorrichtung (1) zur Sammlung des Urins des Patienten, wobei die Urinsammelvorrichtung (1) für die Anbringung auf einer Toilettenschüssel ausgestattet ist;
- Mitteln (12) zur Messung der Daten; und
- einer Verarbeitungseinheit (14), die mit Speichermitteln (13) zur Speicherung der Messdaten versehen ist, **dadurch gekennzeichnet, dass**
die Verarbeitungseinheit (14) zum lösbaren Anbringen auf der Sammelvorrichtung (1) angepasst ist.

2. Vorrichtung nach Anspruch 1, wobei die Sammelvorrichtung (1) mit einer Abdeckung (18) versehen ist, um zumindest teilweise die Verarbeitungseinheit (14) aufzunehmen.

3. Vorrichtung nach Anspruch 1 oder 2, wobei die Messmittel einen Durchflusssensor (12) zur Messung von Daten in Bezug auf den Urinfluss des Patienten umfassen.

4. Vorrichtung nach Anspruch 3, wobei der Durchflusssensor (12) in die Verarbeitungseinheit (14) integriert ist.

5. Vorrichtung nach irgendeinem der vorangehenden Ansprüche, ferner umfassend eine Schnittstelle (16) für die Verbindung mit der Verarbeitungseinheit (14) zur Anzeige der Messdaten.

6. Vorrichtung nach Anspruch 5, wobei die Verarbeitungseinheit (14) ferner mit einem Sender (15) für die drahtlose Übertragung der Messdaten an die Schnittstelle (16) versehen ist, wobei diese Schnittstelle mit einem Empfänger (17) zum Empfang der Messdaten versehen ist.

7. Vorrichtung nach Anspruch 5 oder 6, wobei die Schnittstelle (16) einen Taschencomputer wie z.B. einen PDA, d.h. einen Personal Digital Assistant, umfasst.

8. Vorrichtung nach irgendeinem der vorangehenden Ansprüche, wobei die Urinsammelvorrichtung (1) trichterförmig und mit Mitteln (4, 4A, 5, 5A, 6) zur Anbringung in der Toilettenschüssel versehen ist.

9. Vorrichtung nach irgendeinem der vorangehenden Ansprüche, wobei die Urinsammelvorrichtung (1) eine zumindest teilweise doppelwändige (2, 3) Form annimmt und mit einer Flüssigkeitssperre (7, 8) versehen ist und wobei die Messmittel derart angeordnet sind, dass sie die Luftverdrängung als Folge der Urinproduktion messen.

## Revendications

1. Dispositif de mesure de données se rapportant à la production d'urine d'un patient pour une application médicale, lequel dispositif est prévu avec :
- un dispositif de collecte (1) pour collecter l'urine du patient, lequel dispositif de collecte (1) d'urine est agencé pour être monté sur une cuvette de toilettes ;
- un moyen (12) pour mesurer les données ; et
- une unité de traitement (14) prévue avec un moyen de mémoire (13) pour mémoriser les données de mesure, **caractérisé en ce que**
l'unité de traitement (14) est adaptée pour être montée de façon amovible sur le dispositif de collecte (1).

2. Dispositif selon la revendication 1, dans lequel le dispositif de collecte (1) est prévu avec un couvercle (18) pour recevoir au moins partiellement l'unité de traitement (14).

3. Dispositif selon la revendication 1 ou 2, dans lequel le moyen de mesure comprend un capteur de flux (12) pour mesurer des données se rapportant au flux d'urine provenant du patient.

4. Dispositif selon la revendication 3, dans lequel le capteur de flux (12) est incorporé dans l'unité de traitement (14).

5. Dispositif selon l'une quelconque des revendications précédentes, comprenant en outre une interface (16) pour une connexion à l'unité de traitement (14) pour afficher les données de mesure.

6. Dispositif selon la revendication 5, dans lequel l'unité de traitement (14) est en outre prévue avec un émetteur (15) pour une transmission sans fil des données de mesure à l'interface (16), cette interface étant prévue avec un récepteur (17) pour recevoir les données de mesure.

7. Dispositif selon la revendication 5 ou 6, dans lequel l'interface (16) comprend un ordinateur de poche, tels qu'un PDA, à savoir un Assistant Numérique Personnel.

8. Dispositif selon l'une quelconque des revendications précédentes, dans lequel le dispositif de collecte (1) d'urine a une forme d'entonnoir et est prévu avec un moyen (4, 4A, 5, 5A, 6) pour un montage dans la cuvette de toilettes.

9. Dispositif selon l'une quelconque des revendications précédentes, dans lequel le dispositif de collecte (1) d'urine prend une forme à paroi au moins partiellement double (2, 3) et est prévu avec un verrou pour liquide (7, 8), et dans lequel le moyen de mesure est agencé pour mesurer le déplacement d'air comme un résultat de la production d'urine.
